# EUROPÄISCHE PATENTSCHRIFT

(11) **EP 1 069 056 B1**
(45) Veröffentlichungstag und Bekanntmachung des Hinweises auf die Patenterteilung: **28.07.2004**
(21) Anmeldenummer: 00111674.8
(22) Anmeldetag: 31.05.2000
(51) Int. Cl.: B65D 77/04, B65D 75/58

(54) **Packung zum Lagern von Sterilgut, Vorrichtung und Verfahren zum Herstellen einer derartigen Packung**
Package for a sterile product and device and method for manufacturing the same
Emballage pour le stockage d'un produit stérile ainsi que dispositif et méthode pour sa fabrication

(30) Priorität: 12.07.1999 DE 19932458
(43) Veröffentlichungstag der Anmeldung: 17.01.2001
(60) Teilanmeldung: 03000678.7
(73) Patentinhaber: Lohmann & Rauscher GmbH, 2525 Schönau/Triesting (AT)
(72) Erfinder: Waldner, Wilhlem Ing., 2525 Schönau/Tr (AT); Leuprecht, Helmut Dr., 1130 Wien (AT); Koch, Reinhard, 53489 Sinzig (DE)
(74) Vertreter: Patentanwälte Leinweber & Zimmermann

(56) Entgegenhaltungen:
- DE-A- 3 638 511
- DE-A- 19 603 476
- DE-A- 19 641 199
- DE-A- 19 737 458
- FR-A- 1 391 971
- US-A- 4 337 862
- US-A- 4 714 595
- US-A- 5 511 358

## Beschreibung

Die Erfindung betrifft eine Packung zum Lagern und Darreichen von Sterilgut nach dem Oberbegriff des Patentanspruchs 1, eine zum Herstellen derartiger Packungen betreibbare Vorrichtung.

Derartige Packungen werden beispielsweise zum Bereitstellen von während einer Operation benötigten Verbandstoffen eingesetzt.

Dazu wird die Außenverpackung üblicherweise von einer unsterilen Person geöffnet. Aus der so geöffneten Außenverpackung entnimmt dann eine sterile Person die Innenverpackung, öffnet diese und entnimmt daraus das Sterilgut, wie etwa die benötigten Verbandstoffe.

Nach Abschluß einer Operation und vor Verschluß der Operationswunde muß durch eine Zählkontrolle sichergestellt werden, daß alle benutzten Verbandstoffe und sonstigen Sterilgüter aus dem Körper des Patienten entfernt worden sind. Zu diesem Zweck sind Packungen der eingangs beschriebenen Art üblicherweise mit zumindest den Inhalt der Packung angebenden Zählkarten ausgestattet. Nach den einschlägigen Vorschriften ist es erforderlich, daß die abschließende Zählkontrolle sowohl von einer sterilen Person im Operationssaal, als auch von einer unsterilen Person außerhalb des Operationssaals durchgeführt wird, bevor die Operationswunde verschlossen werden darf. Aus diesem Grund sind in jeder Packung der eingangs angegebenen Art mindestens zwei Zählkarten enthalten.

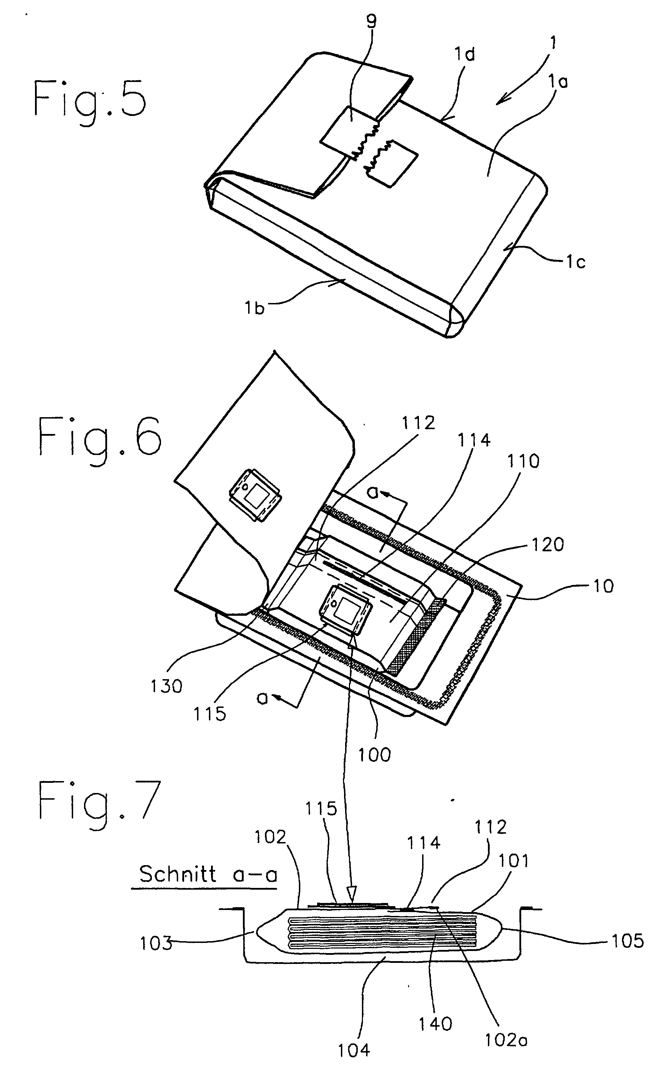

Eine mit diesen beiden Zählkarten ausgestattete Innenverpackung einer Packung der eingangs angegebenen Art nach dem Stand der Technik ist in Fig. 4 dargestellt. Bei dieser Packung ist auf der Außenseite einer in Form eines mit Klebestreifen verschlossenen Papierbeutels 1 verwirklichten und mit Verbandstoffprodukten befüllten Innenverpackung eine zweiteilige Zählkarte mit einem kreisförmigen Klebeelement 2 befestigt. Die Zählkarte selbst besteht aus einer zweiteiligen Klebeetikette 4, 5 mit einem den Inhalt der Innenverpackung darstellenden Aufdruck 8 und einem Trägerstreifen 3 aus nichtklebendem Silikonpapier, auf dem die Klebeetiketten 4, 5 angebracht sind. Die beiden Klebeetiketten 4, 5 und der Trägerstreifen 3 sind mit einer Perforation 7 versehen. Diese Perforation bildet eine Trennlinie, welche die Trennung der einzelnen Klebeetiketten voneinander ermöglicht. Weiter ist in Fig. 4 erkennbar, daß jede der Klebeetiketten 4, 5 mit einer Lochstanzung 6 versehen ist, die zum Aufhängen der gesammelten Zählkarten dient.

Zur Herstellung einer fertigen Packung wird die in Fig. 4 dargestellte Anordnung in eine Außenverpackung (in Fig. 4 nicht dargestellt) eingelegt und sterilisiert. Zur Bereitstellung der in der in Fig. 4 dargestellten Innenverpackung 1 enthaltenen Verbandstoffprodukte während einer Operation wird zunächst die in Fig. 4 nicht dargestellte Außenverpackung von einer unsterilen Person geöffnet. Danach entnimmt eine sterile Person, wie etwa eine OP-Schwester die Innenverpackung 1. Im weiteren Verlauf nimmt die sterile Person die zweiteilige Zählkarte von der Innenverpackung 1 ab, trennt die Zählkarten 4, 5 längs der Trennlinie 7 voneinander und übergibt eines der Zählkartenteile der unsterilen Person.

Dabei muß darauf geachtet werden, daß sowohl die sterile Person als auch die unsterile Person die zu übergebende Zählkarte jeweils an einem ihrer äußeren Enden anfaßt, um so eine Kontaminierung der sterilen Person zu vermeiden. Nicht verhindert werden kann dabei jedoch, daß die unsterile Person der sterilen Person während der Übergabe sehr nahe kommt. Daher ist die Übergabe der Zählkarte an die unsterile Person mit einer erheblichen Kontaminationsgefahr verbunden, die nur bei einer äußerst sorgfältigen Handhabung auf einem noch akzeptablen Maß gehalten werden kann. Ein weiteres Problem bei der Benutzung von Packungen der in Fig. 4 dargestellten Art ist darin zu sehen, daß beim Nachreichen von Verbandstoffen, was beispielsweise bei einem unerwarteten Operationsverlauf notwendig werden kann, der oben bereits erläuterte Vorgang des Öffnens der Außenverpackung und der Entnahme der Innenverpackung wiederholt werden muß, wobei die sterile Person jedoch bereits durch Blut des zu operierenden Patienten kontaminiert ist. Daher besteht hier die Gefahr, daß die unsterile Person bei der Übergabe der Zählkarte beschmutzt bzw. kontaminiert wird. Ferner steht der unsterilen Person nach Übergabe der Zählkarte nur eine kontaminierte bzw. blutige Zählkarte für die Kontrolle und Dokumentation zur Verfügung. Um das zu verhindern, bedarf es derzeit eines sehr aufwendigen Handlings.

Eine Packung mit zwei den Inhalt einer sterilen Innenverpackung darstellenden Informationsträgern, von denen einer im sterilen Innenraum einer Außenverpackung angeordnet ist, ist in der FR 1 391 971 A angegeben. Ferner ist eine Packung mit den Merkmalen des Oberbegriffs des Patentanspruchs 1 in der US 4 714 595 A beschrieben.

Angesichts der vorstehend erläuterten Probleme im Stand der Technik liegt der Erfindung die Aufgabe zugrunde, eine Packung der eingangs beschriebenen Art bereitzustellen, bei deren Benutzung die erforderliche Zählkontrolle ohne Kontaminationsgefahr auf einfache Weise durchgeführt werden kann.

Erfindungsgemäß wird diese Aufgabe durch die im kennzeichnenden Teil des Patentanspruchs 1 angegebene Weiterbildung der bekannten Packung gelöst.

Eine die gewünschte Erfassung durch eine unsterile Person ohne Kontamination der Innenverpackung ermöglichende Anordnung eines Informationsträgers kann besonders einfach verwirklicht werden, wenn dieser Informationsträger lösbar an der Außenverpackung befestigt ist.

Bei der erfindungsgemäßen Packung kann eine Kontamination der Innenverpackung während der Entnahme der Zählkarte durch die unsterile Person besonders sicher ausgeschlossen werden, wenn der von der unsterilen Person zu erfassende Informationsträger lösbar an einer dem sterilen Innenraum zugewandten Innenseite eines Abdeckbereichs der Außenverpackung befestigt ist. Dazu wird zweckmäßigerweise eine Außenverpackung mit einer tiefgezogenen Schale und einem in Form einer darauf aufgesiegelten Papierbahnen verwirklichten Abdeckbereich eingesetzt.

Bei dieser Ausführungsform kann der von der sterilen Person zu benutzende Informationsträger in üblicher Weise an einer Außenseite der sterilen Innenverpackung befestigt werden. Wie vorstehend bereits im Zusammenhang mit Verpackungen nach dem Stand der Technik erläutert, hat es sich als besonders zweckmäßig erwiesen, wenn mindestens einer der Informationsträger einen mit einem den Inhalt der sterilen Innenverpackung angegebenden Aufdruck versehenes Klebeetikett aufweist. Ein derartiges Klebeetikett kann nach Abschluß der Operation auf ein Operationsprotokoll aufgeklebt werden, in dem der Verlauf der Operation sowie eine Dokumentation über die während der Operation eingesetzten Verbandstoffe protokolliert ist.

Dabei kann eine Beschädigung der Zählkarten bei der Abnahme von der sterilen Innenverpackung bzw. des Abdeckbereichs der Außenverpackung zuverlässig verhindert werden, wenn die Klebefläche mindestens einer der Klebeetiketten zumindest teilweise von einer nichtklebenden Abdeckung, wie etwa einem nichtklebenden Silikonpapier abgedeckt ist. Falls die Abdeckung nur einen Teil der Klebefläche des Klebeetikettes abdeckt, kann das Klebeetikett an dem nicht abgedeckten Teil der Klebefläche an der Innenverpackung bzw. der Außenverpackung befestigt werden. Zur Sicherstellung einer beschädigungsfreien Abnahme der Klebeetiketten von der Innenverpackung bzw. der Außenverpackung ist es dabei besonders zweckmäßig, wenn mindestens eine der Klebeetiketten eine vorzugsweise außerhalb der Abdeckung angebrachte Trennlinie, wie etwa eine Perforation aufweist. In besonders vorteilhafter Ausgestaltung der Erfindung weist jede der Klebeetiketten zwei parallel zueinander verlaufende Trennlinien auf, zwischen denen die Abdeckung und der den Inhalt der Innenverpackung angebende Aufdruck angebracht ist. Dann kann die Klebeetikette an zwei Stellen auf die Innenverpackung bzw. auf die Außenverpackung aufgeklebt werden und der zwischen diesen beiden Klebestellen liegende und mit den den Inhalt der Packung angebenden Aufdruck versehene Bereich kann einfach abgenommen werden.

Wie der vorstehenden Erläuterung einer erfindungsgemäßen Packung bereits entnommen werden kann, ist eine erfindungsgemäße Vorrichtung zum Herstellen einer derartigen Packung mit einer Einrichtung zum Verschließen einer die sterile Innenverpackung enthaltenden Außenverpackung und einem zum Befestigen von Informationsträgern an der sterilen Innenverpackung betreibbare Befestigungseinrichtung ausgestattet und im wesentlichen dadurch gekennzeichnet, daß die Befestigungseinrichtung auch noch zum Befestigen von Informationsträgern an der ihrem Innenraum zugewandten Innenseite der Außenverpackung betreibbar ist.

Dazu weist die Verschließeinrichtung der erfindungsgemäßen Vorrichtung zweckmäßigerweise eine erste zum Fördern der Außenverpackung längs einer vorgegebenen Bahn betreibbare Fördereinrichtung und eine zweite zum Fördern einer Abdeckfolie für die Außenverpackung längs der vorgegebenen Bahn betreibbare Fördereinrichtung auf, wobei die zweite Fördereinrichtung eine zum Umlenken der Abdeckfolie um mindestens 90° vorzugsweise etwa 180° um eine senkrecht zu der vorgegebenen Bahn verlaufende Umlenkachse dienende Umlenkeinrichtung aufweist. Durch diese Ausgestaltung der zweiten Fördereinrichtung wird erreicht, daß die die sterile Innenverpackung enthaltende Außenverpackung, wie etwa eine tiefgezogene Schale vor Anbringen der Abdeckfolie offen und von außen zugänglich ist, um so das Anbringen der Informationsträger an den in die Außenverpackungen eingelegten Innenverpackungen zu ermöglichen. Ferner wird durch diese Anordnung der zweiten Fördereinrichtung erreicht, daß die nach Fertigstellung der Packung dem sterilen Innenraum zugewandte Seite der Abdeckfolie vor der Umlenkung frei zugänglich ist und ebenfalls mit einem Informationsträger ausgestattet werden kann.

Zur vollautomatischen maschinellen Fertigung erfindungsgeinäßer Packungen weist die Befestigungseinrichtung der erfindungsgemäßen Vorrichtung in besonders vorteilhafter Ausgestaltung der Erfindung eine zum Zuführen der Informationsträger in einer etwa senkrecht zur vorgegebenen Bahn verlaufenden Zuführrichtung betreibbare Zuführeinrichtung sowie ein Befestigungselement, wie etwa einen in einer senkrecht zu der durch die Zuführrichtung und die vorgegebene Bahn aufgespannten Ebene verlaufenden Aufdruckrichtung hin und her bewegbaren Stempel, auf. Durch einen entsprechend getakteten Betrieb der ersten und zweiten Fördereinrichtung sowie der Zuführeinrichtung und des Befestigungselementes können die erfindungsgemäßen Packungen vollautomatisch hergestellt werden. Dabei kann die Zuführeinrichtung zum Zuführen von auf der Innenverpackung anzubringenden Informationsträgern und auf der der Innenverpackung zugewandten Innenseite der Abdeckfolie anzubringenden Informationsträgern eingesetzt werden, wenn die Zuführeinrichtung und/oder das Befestigungselement in einer etwa parallel zu der vorgegebenen Bahn verlaufenden Richtung hin- und hergehend bewegbar sind. Auf diese Weise kann die Zuführeinrichtung und/oder das Befestigungselement in einer ersten Stellung längs der vorgegebenen Bahn zum Anbringen der Informationsträger auf den in den noch nicht verschlossenen Außenverpackungen aufgenommen Innenverpackungen und in einer durch Verschieben längs der vorgegebenen Bahn erreichten zweiten Stellung zum befestigen von Informationsträgern auf der Abdeckfolie vor deren Umlenkung benutzt werden.

Ein besonders störungsfreier Betrieb der erfindungsgemäßen Vorrichtung zum Befestigen von in Form von Klebeetiketten hergestellten Informationsträgern auf der Innenverpackung und der Außenverpackung kann erreicht werden, wenn die Zuführeinrichtung zum Zuführen von auf einer nichtklebenden Trägerfolie, wie etwa Silikonpapier angebrachten Klebeetiketten ausgelegt ist und mindestens eine Abzieheinrichtung zum Abziehen mindestens eines etwa parallel zur Zuführrichtung verlaufenden Randbereichs der Trägerfolie von den Klebeetiketten umfaßt. Nach Abziehen dieses Randbereiches können die Klebeetiketten dann mit ihrer so freigelegten Klebefläche an der Innenverpackung bzw. der Abdeckfolie der Außenverpackung befestigt werden. Wie vorstehend bereits erläutert, ist es besonders zweckmäßig, wenn die Klebeetiketten mit zwei parallel zueinander verlaufenden Klebeflächen auf der Innenverpackung bzw. der Abdeckfolie der Außenverpackung befestigt sind, wobei ein mit einem den Inhalt der Packung angebenden Aufdruck versehener Bereich der Klebeetiketten zwischen diesen freiliegenden Klebestreifen angeordnet ist und auf seiner dem Aufdruck abgewandten Seite eine noch mit der Abdeckfolie abgedeckte Klebefläche umfaßt.

Mit der erfindungsgemäßen Vorrichtung kann eine besonders hohe Produktionsgeschwindigkeit erreicht werden, wenn die Befestigungseinrichtung zum gleichzeitigen Festlegen von mindestens zwei Informationsträgern auf mindestens zwei Innenverpackungen und/oder mindestens zwei Außenverpackungen betreibbar ist und/oder die erste Fördereinrichtung zum Fördern von mindestens zwei in einer senkrecht zu der vorgegebenen Bahn verlaufenden Richtung nebeneinander angeordneten Außenverpackungen ausgelegt ist.

Wie der vorstehenden Erläuterung einer erfindungsgemäßen Vorrichtung zum Herstellen erfindungsgemäßer Packungen zu entnehmen ist, umfaßt ein erfindungsgemäßes Verfahren zum Herstellen erfindungsgemäßer Packungen unter Verwendung dieser Vorrichtung im wesentlichen das Einlegen von Innenverpackungen in eine entsprechende Außenverpackung, das Anbringen eines Informationsträgers an der in die Außenverpackung eingelegten Innenverpackung sowie das Verschließen der Außenverpackung und ist im wesentlichen dadurch gekennzeichnet, daß zusätzlich auch noch ein Informationsträger an der ihrem Innenraum zugewandten Innenseite der Außenverpackung angebracht wird.

Grundsätzlich sind auch herkömmliche Innenverpackungen der in Fig. 4 dargestellten Art zum Herstellen erfindungsgemäßer Packungen geeignet. Fig. 5 zeigt eine andere perspektivische Ansicht dieser herkömmlichen Innenverpackungen. Danach weist die in Form eines Papierbeutels 1 gebildete Innenverpackung eine erste im wesentlichen rechteckige und der Form des darin aufgenommenen Sterilgutes entsprechende Hauptfläche 1a auf, die über Randabschnitte 1b, 1c und 1d mit einer gegenüberliegenden Hauptfläche des Papierbeutels 1 verbunden ist. Der Papierbeutel ist nur einseitig längs des Randabschnittes 1c verschlossen. Längs seines parallel zu diesem Randabschnitt 1c verlaufenden Randes weist der Papierbeutel 1 eine Öffnung auf. Dieser Randabschnitt ist auf die Hauptfläche 1a zurückgefaltet und, wie vorstehend bereits erläutert, mit einem Klebestreifen 9 auf der Hauptfläche 1a befestigt, wodurch gleichzeitig ein sicherer Verschluß des Papierbeutels 1 erfolgt. Nach der eingangs bereits erläuterten Entnahme der Innenverpackung 1 aus einer Außenverpackung wird die Innenverpackung geöffnet und der Inhalt der Innenverpackung kontrolliert. Dazu wird der Klebestreifen 9 aufgerissen bzw. von der Hauptfläche 1a getrennt. Durch die dann freiwerdende Öffnung des Papierbeutels längs dessen parallel zu dem querverlaufenden Rand 1c verlaufenden Rand kann das Sterilgut kontrolliert bzw. aus dem Papierbeutel 1 entnommen werden.

Es hat sich jedoch gezeigt, daß es im besonderen in der Hektik einer Operation bei der Entnahme des Sterilgutes aus den bekannten Innenverpackungen häufig zu einer Beschädigung bzw. zum Einreissen der die Innenverpackung bildenden Papierhülle kommt. Dadurch kann eine vorzeitige Kontaminierung des in der Innenverpackung aufgenommenen Sterilgutes erfolgen.

Angesichts dieser Probleme im Stand der Technik ist die Innenverpackung der erfindungsgemäßen Packung zweckmäßigerweise so beschaffen, daß die das Sterilgut aufnehmende, vorzugsweise in Form eines zumindest teilweise beschichteten Papierbeutels vorliegende Hülle der Innenverpackung zum Bilden der Entnahmeöffnung im Bereich der Hauptfläche vorbereitet ist.

Diese Weiterbildung geht auf die Erkenntnis zurück, daß die im Stand der Technik auftretenden Probleme im wesentlichen darauf zurückzuführen sind, daß der Randbereich des Papierbeutels nur eine verhältnismäßig geringe Öffnungsfläche zur Verfügung stellt. Aus diesem Grund kann es beim Eingreifen in den Papierbeutel zur Entnahme des Sterilgutes daraus sehr leicht zu einer Beschädigung des Papierbeutels und einer vorzeitigen Kontaminierung des Sterilgutes kommen, wobei im besonderen die beim Aufreissen entstehenden und sich auf dem Sterilgut ablagernden Papierstäube zu Komplikationen, wie etwa Verkapselungen nach der Operation führen können.

Im Gegensatz dazu ist die Hülle der im Rahmen der Erfindung vorzugsweise eingesetzten Innenverpackung zur Bildung einer Entnahmeöffnung im Bereich ihrer Hauptflächen vorbereitet. In diesem Bereich kann eine wesentlich größere Entnahmeöffnung gebildet werden. Dadurch wird selbst in der Hektik einer Operation eine Entnahme des Sterilgutes aus der Innenverpackung ermöglicht, ohne daß es zu Kontaminierungen mit Papierstäuben o. dgl. kommt.

Bei einer besonders bevorzugten Ausführungsform der Erfindung ist die Hülle zum Bilden einer sich ausgehend von der Hauptfläche bis über einen daran angrenzenden Rand des darin aufgenommenen Sterilgutes hinweg erstreckenden Entnahmeöffnung vorbereitet. Mit einer so vorbereiteten Innenverpackung wird eine besonders leichte Überprüfbarkeit des Inhaltes der Innenverpackung bzw. eine besonders einfache Stückzahlkontrolle durch Sicht auf die Falzkanten der in der Innenverpackung aufgenommenen Produkte, wie etwa Kompressen ermöglicht, weil die sich über den Rand des Sterilgutes erstreckende Entnahmeöffnung eine freie Sicht auf diese Falzkanten erlaubt. Im übrigen wird auf diese Weise auch noch ein besonders einfacher Zugriff auf das in der Innenverpackung aufgenommene Produkt ermöglicht. Dabei kann eine kontaminationsfreie Übergabe dieses Produktes erreicht werden, wenn die Hauptfläche auch nach Bildung der Entnahmeöffnung noch von einem Teil der Hülle abgedeckt wird. In diesem Fall kann das in der Innenverpackung aufgenommene Produkt durch die starre, nicht zum Öffnen der Innenverpackung benötigte Hand fixiert werden, in dem es über die noch daran anliegende Hülle in der Innenverpackung festgehalten wird, während der im Bereich der Entnahmeöffnung freiliegende Rand des Sterilgutes ohne Behinderung durch die das Sterilgut in der Innenverpackung haltende Person erfaßt werden kann.

Ähnlich wie bei der anhand der Fig. 5 erläuterten bekannten Innenverpackung kann auch die Hülle der im Rahmen der Erfindung bevorzugten Innenverpackung eine das Sterilgut zumindest teilweise umlaufende Materialbahn aufweisen. In diesem Fall hat es sich zur Bildung der im Bereich der Hauptfläche der Innenverpackung angeordneten Entnahmeöffnung als besonders günstig erwiesen, wenn diese Materialbahn einen ersten Abschnitt und einen zweiten Abschnitt aufweist, von denen jeder einen Teil der Hauptfläche bildet, wobei sich diese Abschnitte im Bereich der Hauptfläche teilweise überlappen. Dann wird einerseits eine vollständige Abdeckung des Sterilgutes durch die Materialbahn im Bereich der Hauptfläche erreicht, während andererseits eine besonders einfache Öffnung im Bereich der Hauptfläche erzeugt werden kann, in dem der erste Abschnitt und der zweite Abschnitt ausgehend von der überlappenden Anordnung voneinander getrennt werden.

Herstellungstechnisch hat es sich als besonders günstig erwiesen, wenn die im Rahmen der Erfindung bevorzugte Innenverpackung in Form eines aus einer Materialbahn gebildeten Schlauchbeutels hergestellt ist, wobei die Materialbahn einen ersten, einen Teil der Hauptfläche bildenden Abschnitt, einen zweiten einen weiteren Teil der Hauptfläche bildenden Abschnitt, einen dritten eine erste Umlaufachse teilweise umlaufenden Abschnitt, einen vierten, die der Hauptfläche entgegengesetzte Begrenzungsfläche der Innenverpackung bildenden Abschnitt und einen fünften eine zweite vorzugsweise etwa parallel zu der ersten Umlaufachse verlaufende Umlaufachse teilweise umlaufenden Abschnitt aufweist, wobei der dritte Abschnitt einen zwischen dem ersten Abschnitt und dem vierten Abschnitt angeordneten Randbereich der Innenverpackung bildet und der fünfte Abschnitt einen wieteren zwischen dem vierten Abschnitt und dem zweiten Abschnitt angeordneten Randbereich der Innenverpackung bildet.

Bei dieser Anordnung kann die Hülle besonders einfach zum Bilden der Entnahmeöffnung im Bereich der Hauptfläche vorbereitet werden, wenn der erste Abschnitt und der zweite Abschnitt sich längs eines etwa parallel zu den Umlaufachsen bzw. in Längsrichtung des Schlauchbeutels verlaufenden Überlappungsbereiches überlappen, wobei diese Abschnitte die in Längsrichtung des Schlauchbeutels verlaufenden Ränder der Materialbahn bilden.

Zur Stabilisierung der im Rahmen der Erfindung bevorzugten Innenverpackung hat es sich als besonders günstig erwiesen, wenn der erste Abschnitt und der zweite Abschnitt in dem Überlappungsbereich lösbar miteinander verbunden, insbesondere verklebt oder versiegelt sind. Dabei kann sowohl eine Kaltsiegelung als auch eine Heißsiegelung zum Einsatz kommen. Zur Vermeidung einer Kontamination des in der Innenverpackung aufgenommenen Sterilgutes wird die Haltekraft der Verbindung zweckmäßigerweise so gering gewählt, daß beim Lösen der Verbindung keine Beschädigung des ersten Abschnittes oder des zweiten Abschnittes erfolgt.

Wenn der zweite Abschnitt auf seiner dem Sterilgut zugewandten Seite mit dem ersten Abschnitt verbunden ist und in seiner sich senkrecht zu den Umlaufachsen erstreckenden Richtung eine die Hälfte der Breite der Hauptfläche überschreitende Breite aufweist, kann die Entnahmeöffnung durch Abheben des zweiten Abschnittes von dem ersten Abschnitt gebildet werden, wobei der erste Abschnitt eine besonders große Fläche zum Anbringen des beispielsweise in Form eines Klebeetiketts gebildeten Informationsträgers zur Verfügung stellt. Bei dieser Ausführungsform der Erfindung kann der Informationsträger also im Bereich des ersten Abschnittes auf der Hauptfläche der Innenverpackung angeordnet werden, ohne daß dadurch die Bildung der Entnahmeöffnung beeinträchtigt wird, während gleichzeitig eine besonders einfache Entfernung des Informationsträgers von der Innenverpackung ohne Kontaminierung des Sterilgutes möglich ist, weil der den Informationsträger tragende Abschnitt der Innenverpackung zunächst von dem ersten Abschnitt bzw. dem Sterilgut abgehoben werden kann und erst in diesem Zustand die Abnahme des Informationsträgers erfolgt. Durch diese Anordnung wird also eine dezentrale Positionierung des Informationsträgers ermöglicht, so daß dieser die Längsnaht des Schlauchbeutels nicht überlappt.

Hinsichtlich der Fixierung des in der geöffneten Innenverpackung aufgenommenen Sterilgutes mit der starren, nicht öffnenden Hand hat es sich als besonders günstig erwiesen, wenn der erste Abschnitt längs einer parallel zum Überlappungsbereich verlaufenden Linie auf sich selbst zurückgefaltet ist, so daß ein Haltebereich doppelter Materialstärke im Bereich der Hauptfläche bereitgestellt wird. Dabei kann ein besonders großer Haltebereich bereitgestellt werden, wenn auch der erste Abschnitt in einer sich senkrecht zu den Umlaufachsen verlaufenden Richtung eine die Hälfte der Breite der Hauptfläche überschreitende Breite aufweist.

Die Bereitstellung eines Haltebereichs doppelter Materialstärke wird bei der zuletzt beschriebenen Ausführungsform dadurch erreicht, daß der auf sich selbst zurückgefaltete Teil des ersten Abschnitts nicht vollständig von dem zweiten Abschnitt überlappt wird, so daß ein Rand des zweiten Abschnittes auch dann noch erfaßt werden kann, wenn das Sterilgut in der Innenverpackung im Bereich des auf sich selbst zurückgefalteten ersten Abschnittes erfaßt wird. Derartige Innenverpackungen sind besonders gut für verhältnismäßig große Produkte, wie etwa sterile Kompressen geeignet. Daneben kann eine im Rahmen der Erfindung bevorzugte Innenverpackung aber auch für kleine Produkte oder nebeneiander darin angeordnete Produkte benutzt werden. Bei Einsatz der bevorzugten Innenverpackung für besonders kleine Produkte ist es häufig nicht möglich, die Innenverpackung mit einer Hand zu halten und mit der anderen Hand zu öffnen, weil nicht genügend Griffraum für die haltende Hand zur Verfügung steht. In diesem Fall ist es besonders günstig, wenn die Innenverpackung mit beiden Händen geöffnet wird. Dazu kann der auf sich selbst zurückgefaltete Teil des ersten Abschnittes vollständig von dem zweiten Abschnitt überlappt werden. Die Öffnung der Innenverpackung erfolgt in diesem Fall so, daß die Innenverpackung auf einer Unterlage abgelegt wird, der auf sich selbst zurückgefaltete Teil des ersten Abschnittes zusammen mit dem diesen überlappenden Teil des zweiten Abschnittes hochgeklappt wird, dann die freiliegenden Ränder dieser Abschnitte mit jeweils einer Hand erfaßt und voneinander getrennt werden. Diese Handhabung ist auch dann von besonderem Vorteil, wenn in der Innenverpackung nebeneinander angeordnete Produkte aufgenommen sind, weil dann die ansonsten bestehende Gefahr beseitigt ist, daß das nicht festgehaltene sterile Gut während des Öffnungsvorgangs aus der Innenverpackung herausfällt.

Bei der Öffnung der Innenverpackung kann die Gefahr einer Beschädigung der im allgemeinen aus Papier bestehenden Hülle weiter verringert werden, wenn ein freiliegender Rand des ersten Abschnitts und/oder des zweiten Abschnittes, also im allgemeinen die Längskante der Materialbahn zur Bildung einer verstärkten Anfaßlasche zumindest teilweise auf sich selbst zurückgefaltet ist.

Wie eingangs bereits erläutert wird die Innenverpackung vorzugsweise in Form eines Schlauchbeutels verwirklicht. Dazu ist der erste Abschnitt und/oder der zweite Abschnitt längs mindestens eines quer zu den Umlaufachsen verlaufenden Verbindungsbereiche mit dem vierten Abschnitt verbunden, insbesondere verklebt oder versiegelt. Zweckmäßigerweise erfolgt die Verbindung längs beider senkrecht zu den Umlaufachsen verlaufenden Ränder des ersten Abschnittes und des zweiten Abschnittes. Zur Vermeidung einer die Erzeugung von Papierstaub verursachenden Beschädigung der Innenverpackung ist auch die Haltekraft dieser Verbindung vorzugsweise so gering, daß beim Lösen dieser Verbindung keine Beschädigung des ersten, zweiten oder vierten Abschnittes erfolgt.

Die Gefahr einer unbeabsichtigten Beschädigung der Innenverpackung kann weiter verringert werden, wenn sich die Verbindung des ersten Abschnittes mit dem zweiten Abschnitt nicht mit der Verbindung des ersten bzw. zweiten Abschnittes mit dem vierten Abschnitt kreuzt, weil ein Einreissen des Papiers oder Ablösen von Papierteilchen beim Öffnen der Innenverpackung vorzugsweise an derartigen Kreuzungspunkten auftritt. Zur Vermeidung derartiger Kreuzungspunkte wird besonders bevorzugt die Verbindung des ersten Abschnittes mit dem zweiten Aschnitt nicht über die gesamte Länge des Verbindungsbereiches ausgeführt, weil so gleichzeitig eine leichtere Handhabung beim Öffnen ermöglicht wird.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- Fig. 1: eine perspektivische Ansicht einer erfindungsgemäßen Packung
- Fig. 2: eine schematische Seitenansicht einer Vorrichtung zum Herstellen der in Fig. 1 dargestellten Packung,
- Fig. 3: eine schematische Draufsicht auf die in Fig. 2 dargestellte Vorrichtung,
- Fig. 4: eine perspektivische Ansicht einer Innenverpackung einer Packung zum Lagern von Sterilgut nach dem Stand der Technik,
- Fig. 5: eine perspektivische Ansicht einer zum Herstellen einer erfindungsgemäßen Packung geeigneten herkömmlichen Innenverpackung,
- Fig. 6: eine perspektivische Ansicht einer in einer Außenverpackung aufgenommenen Innenverpackung,
- Fig. 7: eine Schnittansicht längs der in Fig. 6 angegebenen Schnittebene a-a,
- Fig. 8: und 9 Darstellungen zur Erläuterung der Handhabung der in den Fig. 6 und 7 gezeigten Innenverpackung,
- Fig. 10: eine Schnittdarstellung einer Innenverpackung gemäß einer weiteren Ausführungsform der Erfindung,
- Fig. 11: eine Schnittdarstellung einer zum Aufnehmen kleiner oder nebeneinander angeordneter Produkte geeigneten Innenverpackung und
- Fig. 12 und 13: Darstellungen zur Erläuterung der Handhabung der in Fig. 11 dargestellten Innenverpackung.

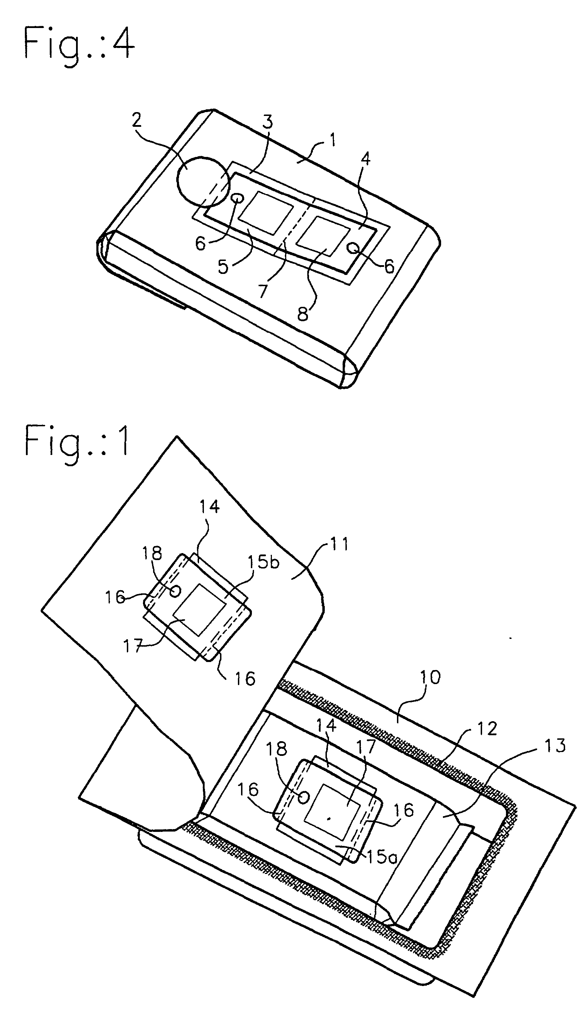

Die in Fig. 1 dargestellte Packung umfaßt eine Innenverpackung 13 in Form eines maschinell befüllten und verschlossenen Papierschlauchbeutels 13 sowie eine in Form einer tiefgezogenen Schale 10 mit einer aufgesiegelten Papierdeckbahn 11 gebildete Außenverpackung. Bei der Herstellung dieser Packungen wird der Papierschlauchbeutel 13 in einer Verpackungsmaschine in die tiefgezogene Schale 10 eingelegt. Vor dem Aufsiegeln der Papierdeckbahn 11 werden Zählkarten getrennt voneinander einerseits auf den Papierschlauchbeutel 13 und andererseits an der dem Innenraum der tiefgezogenen Schale 10 zugewandten Seite der Papierdeckbahn 11 angebracht.

Dabei umfaßt jede der Zählkarten ein mit einem Aufdruck 17 versehenes Klebeetikett 15a und 15b, deren dem Papierschlauchbeutel 13 bzw. der Innenseite der Papierdeckbahn 11 zugewandte Klebefläche teilweise von einer Trägerfolie 14 abgedeckt ist. Jede der Klebeetiketten 15a und 15b umfaßt jedoch parallel zueinander verlaufende und beidseits der nichtklebenden Trägerfolie angeordnete Klebeflächen, von denen jede mit einer parallel zu einem Rand der Trägerfolie 14 verlaufenden Perforation 16 versehen ist. Mit diesen Klebeflächen können die Klebeetiketten auf dem Papierschlauchbeutel 13 bzw. die Innenseite der Papierdeckbahn 11 aufgeklebt werden, während die Perforationen 16 ein einfaches, sicheres und beschädigungsfreies Abnehmen der Klebeetiketten 15a und 15b von dem Papierschlauchbeutel 13 bzw. der Papierdeckbahn 11 ermöglichen.

Wie in Fig. 1 weiter dargestellt, weist jede der Klebeetiketten bzw. Zählkarten 15a und 15b auch noch eine Lochstanzung 18 auf, die zum Aufhängen der gesammelten Zählkarten eingesetzt werden kann.

Beim Einsatz der in Fig. 1 dargestellten Packung wird zunächst die aus der tiefgezogenen Schale 10 und Papierdeckbahn 11 bestehende Außenverpackung durch eine unsterile Person geöffnet, in dem die Papierdeckbahn von der tiefgezogenen Schale abgezogen wird. Dieser Zustand ist in Fig. 1 dargestellt. Danach entnimmt eine sterile OP-Schwester den das Sterilgut, wie etwa Verbandstoffprodukte, enthaltenden Papierschlauchbeutel 13. Die OP-Schwester kann dann die Zählkarte bzw. das Klebeetikett von dem Papierschlauchbeutel 13 abnehmen und den Beutelinhalt überprüfen. Andererseits kann die auf der Innenseite der Papierdeckbahn 11 angebrachte Zählkarte 15b ohne Gefahr einer Kontamination des Papierschlauchbeutels 13 von der unsterilen Person von der Innenseite der Papierdeckbahn 11 abgenommen werden, die die Außenverpackung sowieso in den Händen hält. Dadurch wird eine Kontaminationsgefahr sicher ausgeschaltet.

Ein entscheidender Vorteil der gerade erläuterten erfindungsgemäßen Packung ist also darin zu sehen, daß aufgrund der getrennt angebrachten Zählkarten 15 und 15b die sterile und die unsterile Person nicht mehr, wie bei der alten Verpackungsweise mit den Händen nahe zusammenkommen müssen. Dadurch wird die Kontaminationsgefahr über die Zählkarten ausgeschaltet und eine Voraussetzung für eine kontaminationsfreie Dokumentation gewährleistet.

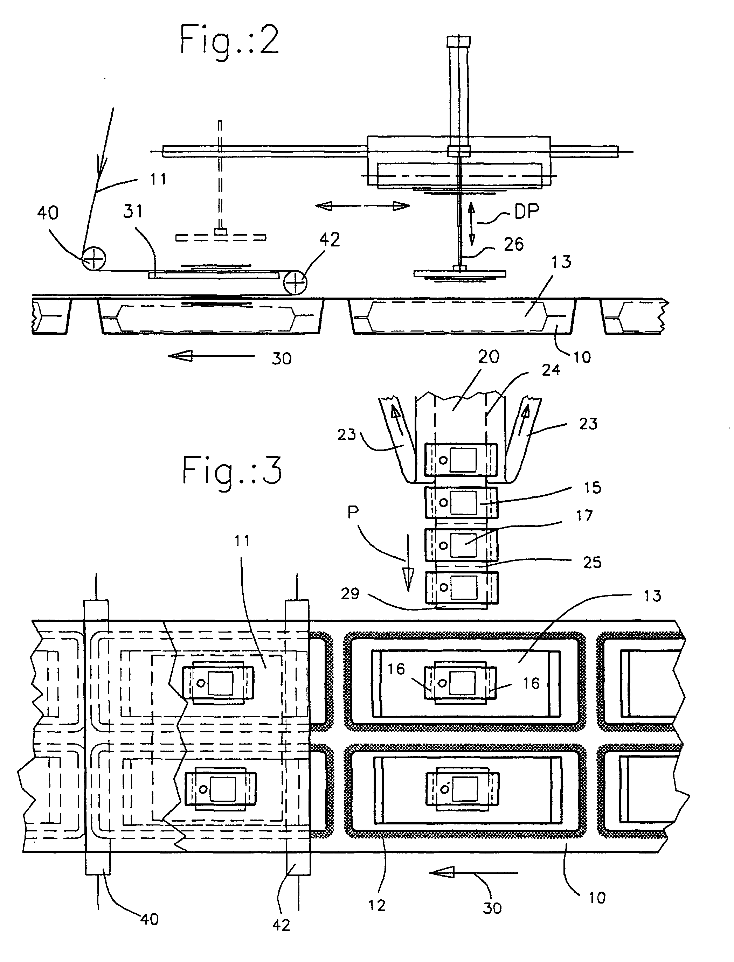

In den Fig. 2 und 3 ist die Anbringung der Zählkarten auf der in Fig. 1 dargestellten Packung vereinfacht dargestellt.

Dazu werden mit den benötigten Informationen, wie etwa dem Packungsinhalt, der Menge, dem Verfallsdatum, der Chargenbezeichnung, notwendigen Prüfzeichen u. dgl., bedruckte Klebeetiketten 15, die auf einer Trägerfolie 20 aus Silikonfolie oder einer anderen nichtklebenden Substanz angebracht sind, benutzt. Dabei sind die Klebeetiketten bereits in einer gewünschten Anordnung positioniert. Der aus der Trägerfolie und den Klebeetiketten bestehende Streifen wird dann getaktet durch eine Zuführeinrichtung in einer durch den Pfeil P bezeichneten Zuführrichtung zugeführt. Gleichzeitig werden während des Zuführvorgangs zwei parallel zur Zuführrichtung P verlaufende Randstreifen 23 der Trägerfolie 20 mit Hilfe einer bereits vorhandenen Perforation 24 abgetrennt. Die danach verbleibende Trägerfolie 29 ist in einer senkrecht zur Zuführrichtung P verlaufenden Richtung schmaler als die Klebeetiketten 15, so daß eine selbstklebende Unterseite der Klebeetiketten 15 beidseitig über die Trägerfolie 29 hinausragt. Die einzelnen Klebeetiketten 15 sind in der Zuführrichtung P voneinander beabstandet. Dadurch ergibt sich ein Überstand der Trägerfolie in der Zuführrichtung P.

Die aus der Trägerfolie 29 und den Klebeetiketten 15 bestehenden Zählkarten werden nun mittels einer in der Zeichnung nicht näher dargestellten Fördereinrichtung über den mit Hilfe einer ersten Fördereinrichtung in einer senkrecht zur Zuführrichtung P verlaufenden und durch den Pfeil 30 bezeichneten Richtung geförderten tiefgezogenen Folien 10 positioniert und durch einen Stempel 26 (vgl. Fig. 2) auf bereits in den tiefgezogenen Schalen 10 angeordneten Papierschlauchbeuteln befestigt. Dazu ist der Stempel 26, wie in Fig. 2 durch den Doppelpfeil DP angedeutet in einer senkrecht zur Förderrichtung der ersten Fördereinrichtung und der Zuführrichtung aufgespannten Ebene verlaufenden Richtung hin- und hergehend bewegbar. Im weiteren Verlauf der Förderung der tiefgezogenen Schalen 10 längs der durch den Pfeil 30 angegebenen vorgegebenen Bahn wird die Öffnung der einzelnen tiefgezogenen Schalen mit einer Papierdeckbahn 11 verschlossen.

Die Papierdeckbahn 11 wird mit einer eine erste Umlenkrolle 40 und eine zweite Umlenkrolle 42 aufweisenden Fördereinrichtung derart zugeführt, daß sie zwischen der ersten Umlenkrolle 40 und der zweiten Umlenkrolle 42 in einer der Förderrichtung 30 der ersten Fördereinrichtung entgegengestetzten Richtung gefördert wird und erst nach Umlaufen der zweiten Umlenkrolle 42 parallel zur Förderrichtung 30 der ersten Fördereinrichtung gefördert wird und auf die tiefgezogene Schale 10 aufgesiegelt werden kann.

In dem zwischen der ersten Umlenkrolle 40 und der zweiten Umlenkrolle 42 liegenden Streckenabschnitt werden die Klebeetiketten auf der im fertiggestellten Zustand dem Innenraum der tiefgezogenen Schale 10 zugewandten Innenseite der Papierdeckbahn 11 angebracht. Dazu ist in dem Zwischenraum zwischen der Papierdeckbahn 11 und den längs der vorgegebenen Bahn geförderten tiefgezogenen Schale im Bereich zwischen der ersten Umlenkrolle 40 und der zweiten Umlenkrolle 42 eine Gegenplatte 31 angebracht, mit der eine Beschädigung der Papierdeckbahn 11 während des Anbringens der Klebeetiketten darauf verhindert werden kann. Wie in Fig. 2 durch den Doppelpfeil 44 angedeutet, ist die zum Zuführen der Klebeetiketten ausgelegte Zuführeinrichtung und der Stempel 26 längs der vorgegebenen Bahn der tiefgezogenen Schalen 10 hin- und hergehend bewegbar. Dadurch wird erreicht, daß die in Fig. 3 dargestellte Befestigungseinrichtung in einer ersten in Fig. 2 mit durchgezogenen Linien dargestellten Position zum Anbringen von Klebeetiketten an den Papierschlauchbeuteln 13 und in einer zweiten in Fig. 2 strichliert dargestellten Position zum Anbringen der Klebeetiketten auf der Innenseite der Papierdeckbahn 11 betrieben werden kann.

Wie besonders deutlich der Fig. 3 zu entnehmen ist, sind die einzelnen Perforationen 16 beidseits, außerhalb und parallel zur Trägerfolie angeordnet. Dadurch kann die nur an den Rändern aufgeklebte Zählkarte leicht mit zwei Fingern gefaßt und von der Packung gelöst werden.

Weiter ist der Fig. 3 zu entnehmen, daß die erfindungsgemäße Vorrichtung zur Fertigstellung von zwei oder mehr parallel zueinander geförderten Packungen ausgelegt sein kann.

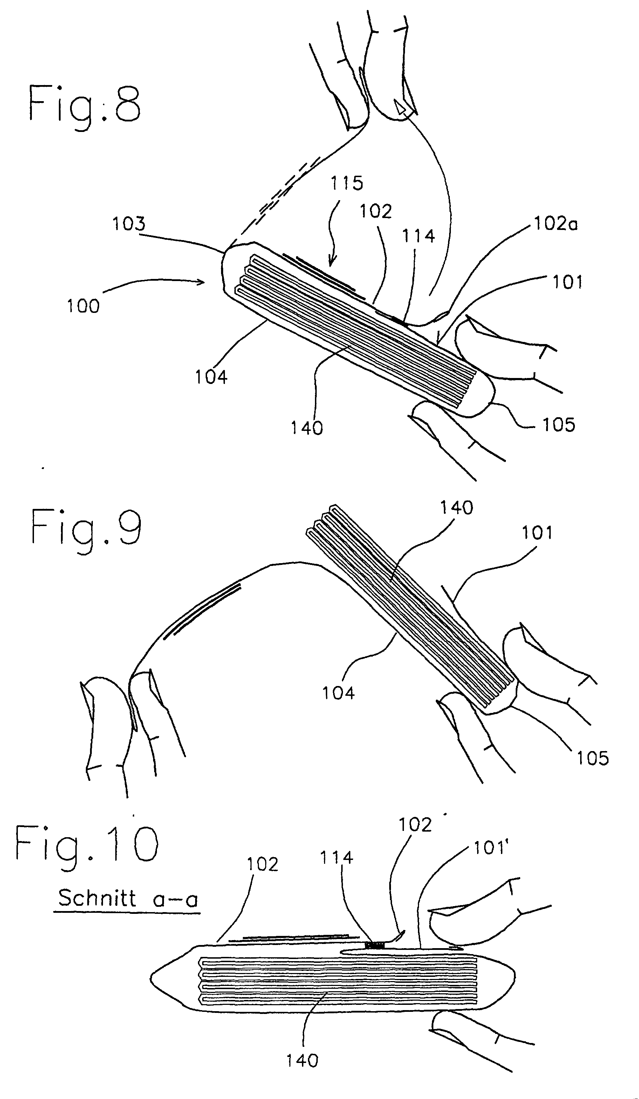

Fig. 6 zeigt eine in einer in Form einer tiefgezogenen Schale 10 ausgeführten Außenverpackung aufgenommene Innenverpackung. Diese Innenverpackung weist eine in Form eines Schlauchbeutels 100 aus Papier gebildete Hülle auf. Diese Hülle 100 bildet eine in Fig. 6 nach oben weisende, im wesentlichen recheckige Hauptfläche 110 auf der ein Informationsträger 115 in der anhand der Fig. 1 erläuterten Weise angebracht ist. Die den Schlauchbeutel 100 bildende Materialbahn weist zwei in Längsrichtung verlaufende Ränder auf, welche sich im Bereich der Hauptfläche 110 überlappen und längs einer Verbindungslinie 114 miteinander verbunden sind. Längs ihrer quer zu der Verbindungslinie 114 bzw. den Längsrändern verlaufenden Querränder sind die einander gegenüberliegenden Abschnitte der den Schlauchbeutel 100 bildenden Materialbahn miteinander versiegelt. Wie besonders deutlich in Fig. 7 zu erkennen ist, weist der Schlauchbeutel 100 einen ersten, einen Teil der Hauptfläche 110 bildenden Abschnitt 101, einen zweiten, einen weiteren Teil der Hauptfläche bildenden Abschnitt 102, einen dritten, einen Rand des in dem Schlauchbeutel aufgenommenen Sterilgutes (hier einen Kompressenstapel) umlaufenden Abschnitt 103, einen vierten die der Hauptfläche entgegengesetzte Begrenzungsfläche der Innenverpackung bildenden Abschnitt 104 sowie einen fünften einen parallel zum dritten Abschnitt verlaufenden Rand umlaufenden Abschnitt 105 auf. Dabei überlappt der zweite Abschnitt 102 im Bereich der Hauptfläche 110 den ersten Abschnitt 101 und weist in einer senkrecht zur Umlaufachse des dritten Abschnitts und des fünften Abschnittes bzw. senkrecht zur Längsrichtung des Schlauchbeutels verlaufenden Richtung eine die Hälfte der Breite der Hauptfläche 110 überschreitende Breite auf. Auf diesem zweiten Abschnitt 102 ist der Informationsträger 115 angebracht.

Zur Herstellung der Verbindung des zweite Abschnittes 102 mit dem ersten Abschnitt 101 ist auf der dem Sterilgut 140 zugewandten Innenseite des zweiten Abschnitts 102 längs der Verbindungslinie 14 eine siegelfähige Beschichtung aufgebracht. Dabei erstreckt sich die Verbindungslinie 114 nur über einen zentralen Teil der gesamten Länge des Schlauchbeuteils 100. Auf diese Weise wird eine Kreuzung der Verbindungslinie 114 mit den Verbindungsbereichen 120 und 130 vermieden. Die Verbindung der einzelnen Abschnitte des Schlauchbeutels im Bereich der Verbindungslinie 114 und der Verbindungslinie 120 und 130 ist so beschaffen, daß die Haltekraft dieser Längs- bzw. Querversiegelung so gering ist, daß eine Lösung der einzelnen Abschnitte voneinander ohne Papierablösung erfolgen kann. Durch die Ausbildung des zweiten Abschnitts 102 mit einer die Hälfte der Breite der Hauptfläche überschreitenden Breite wird die in Fig. 6 und 7 dargestellte dezentrale Positionierung des Informationsträgers auf der Hauptfläche ermöglicht, wobei dieser Informationsträger außerhalb des Überlappungsbereichs 112 des ersten Abschnittes und des zweiten Abschnittes angeordnet ist.

Wie in Fig. 7 angedeutet und besonders deutlich in den Fig. 8 und 9 erkennbar, ist der freiliegende Längsrand 102a des zweiten Abschnittes 102 auf sich selbst zurückgefaltet und bildet so eine verstärkte Anfaßlasche.

Wie anhand der Fig. 8 und 9 zu erkennen ist, wird die in den Fig. 6 und 7 dargestellte Innenverpackung geöffnet, indem das Sterilgut 140 mit einer Hand innerhalb des Schlauchbeutels 100 im Bereich des erste Abschnittes 101 und des vierten Abschnittes 104 festgehalten bzw. fixiert wird, während der zweite Abschnitt 102 mit der anderen Hand im Bereich der Anfaßlasche 102a erfaßt und von dem Sterilgut 140 abgehoben wird, um so eine Entnahmeöffnung zu bilden. Im Verlauf des Öffnungsvorganges wird der zweite Abschnitt 102 zusammen mit dem dritten Abschnitt 103 über den Rand des Sterilgutes 140 in Richtung auf den vierten Abschnitt 104 geklappt, wie in Fig. 9 dargestellt. Auf diese Weise wird eine sich ausgehend von der Hauptfläche 110 über den Rand des Sterilgutes 140 hinweg erstreckende Entnahmeöffnung für das Sterilgut bereitgestellt. Dabei wird durch die Beschaffenheit der Verbindung im Bereich der Verbindungslinie 114 und der Verbindungsbereiche 120 und 130 sichergestellt, daß ein Aufschälen (Peelen) der Verbindung ohne Beschädigung des Materials des Schlauchbeutels erfolgt.

Die in Fig. 9 dargestellte Entnahmeöffnung ermöglicht eine Überprüfung des Produktes und Kontrolle der Stückzahl ohne Berührung der Produkte (z.B. durch bereits kontaminierte Hände während der Operation).

Die in Fig. 10 dargestellte Ausführungsform unterscheidet sich im wesentliche nur dadurch von der anhand der Fig. 6 bis 9 erläuterten Ausführungsform, daß der erste Abschnitt 101' längs einer parallel zum Überlappungsbereich 112 verlaufenden Linie auf sich selbst zurückgefaltet ist und insgesamt ebenfalls eine die Hälfte der Breite der Hauptfläche 110 überschreitende Breite aufweist. Dabei wird der auf sich selbst zurückgefaltete Teil des ersten Abschnitts 101' nur teilweise von dem zweiten Abschnitt 102 überlappt, so daß durch die Zurückfaltung des ersten Abschnittes ein Bereich doppelter Materialstärke zum Festhalten des Sterilgutes 140 in der zu öffnenden bzw. bereits geöffneten Innenverpackung bereitgestellt wird. Weiter ist in Fig. 10 erkennbar, daß der freiligende Rand des ersten Abschnitts 101' ähnlich wie der freiliegende Rand 102a des zweiten Abschnittes 102 zur Bildung einer verstärkten Anfaßlasche auf sich selbst zurückgefaltet ist.

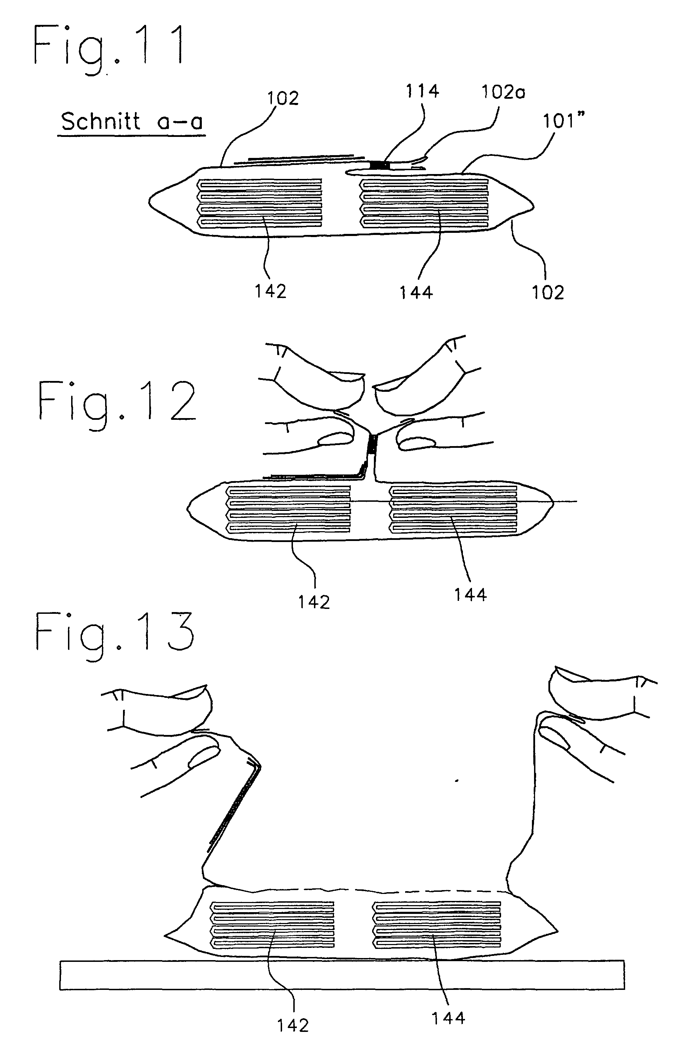

In den Fig. 11 bis 13 ist eine weitere Ausführungsform einer im Rahmen der Erfindung einsetzbaren Innenverpackung dargestellt, die im besonderen zum Aufnehmen von nebeneinander angeordneten Sterilgutstapeln 142 und 144 geeignet ist. Diese Ausführungsform unterscheidet sich nur dadurch von der anhand der Fig. 10 erläuterten Ausführungsform, daß der erste Abschnitt 101'' des Schlauchbeutels 100 derart auf sich zurückgefaltet ist, daß er vollständig von dem zweiten Abschnitt 102 überlappt wird. Zum Öffnen der Innenverpackung wird der auf sich selbst zurückgefaltete Teil des ersten Abschnittes 101'' zusammen mit dem diesen überlappenden Teil des zweiten Abschnitts 102 nach oben geklappt, wie in Fig. 12 dargestellt. Dann werden die freiliegenden Ränder des ersten Abschnittes 101'' und des zweiten Abschnittes 102 mit jeweils einer Hand ergriffen und voneinander getrennt. Auf diese Weise werden die Längs- und Quernähte der Innenverpackung aufgepeelt. Durch die dann entstehende jeweils feste, halbseitige Siegelnaht entsteht eine Tasche, in der das Produkt plaziert bleibt und dargereicht werden kann. Bei allen anhand der Fig. 6 bis 13 erläuterten Ausführungsformen der Erfindung kann durch eine teilweise oder unterschiedlich feste Versiegelung der Quernaht (z.B. 50%ige halbeseitige Versiegelung) der Öffnungs- bzw. Peelvorgang erleichtert werden.

Zur Durchführung der Versiegelung wird das den Schlauchbeutel bildende Papier auf seiner Innenseite mit einer siegelfähigen Beschichtung versehen. Dabei ist die Haltekraft der Längs- und Quersielung jedoch nur so fest, daß sie ohne Papierablösung gelöst werden kann. Wie eingangs bereits erläutert ist bei der anhand der Fig. 6 bis 9 erläuterten Ausführungsform die beschichtete Innenseite mit der unbeschichteten Außenseite der Verpackung verbunden. Bei den anhand der Fig. 10 bis 13 erläuterten Ausführungsformen werden beide Innenseiten beschichtet und miteinander versiegelt.

Als Material für die Innenverpackung können je nach Sterisisationsverfahren teilbeschichtete (Rahmen-) oder vollflächig beschichtete Papiere oder Kunststoffe sowie Verbundpackstoffe in unterschiedlichen Zusammensetzungen, kalt- oder heißsiegelfähig, verwendet werden. Vollbeschichtete Papiere werden z.B. mit γ-Strahlen sterilisiert. Für EO oder Dampfsterisisation sind rahmenbechichtete Papier erforderlich.

Die Erfindung ist nicht auf die anhand der Zeichnung erläuterten Ausführungsformen beschränkt. Vielmehr ist auch an den Einsatz von Packungen gedacht, bei denen die von der nicht sterilen Person zu erfassenden Klebeetiketten im Bereich der tiefgezogenen Schale 10 angebracht sind. Ferner können anstelle von Klebeetiketten auch Informationsträger in Form von Magnetstreifen od. dgl. eingesetzt werden. Darüber hinaus ist auch an den Einsatz von Verpackungsvorrichtungen gedacht, mit denen drei oder mehr parallel zueinander geförderte Packungen fertiggestellt werden können.

## Patentansprüche

1. Packung zum Lagern von Sterilgut mit einer einen sterilen Innenraum aufweisenden Außenverpackung (10, 11), einer das Sterilgut enthaltenden und im sterilen Innenraum der Außenverpackung (10, 11) aufgenommenen sterilen Innenverpackung (13) und mindestens zwei zumindest den Inhalt der Innenverpackung (13) darstellende Informationen tragenden Informationsträgern (15a, 15b), von denen mindestens einer (15a) im sterilen Innenraum der Außenverpackung (10, 11) angeordnet ist, wobei mindestens einer der Informationsträger (15b) beim Vorgang der Entnahme der Innenverpackung (13) aus der Außenverpackung (10, 11) ohne Kontamination der Innenverpackung (13) von einer unsterilen Person erfaßbar ist, **dadurch gekennzeichnet, daß** beide Informationsträger (15a, 15b) in dem sterilen Innenraum der Außenverpackung (10, 11) angeordnet sind.

2. Packung nach Anspruch 1, **dadurch gekennzeichnet, daß** mindestens einer der Informationsträger (15b) an der Außenverpackung (11) befestigt ist.

3. Packung nach Anspruch 1 oder 2, **dadurch gekennzeichnet, daß** mindestens einer der Informationsträger (15b) lösbar an einer dem sterilen Innenraum zugewandten Innenseite eines Abdeckbereichs (11) der Außenverpackung (10, 11) befestigt ist.

4. Packung nach einem der vorhergehenden Ansprüche, **dadurch gekennzeichnet, daß** mindestens einer der Informationsträger (15a) lösbar an einer Außenseite der sterilen Innenverpackung (13) befestigt ist.

5. Packung nach einem der vorhergehenden Ansprüche, **dadurch gekennzeichnet, daß** mindestens einer der Informationsträger (15a, 15b) ein mit einem den Inhalt der sterilen Innenverpackung (13) angebenden Aufdruck (17) versehenes Klebeetikett aufweist.

6. Packung nach Anspruch 5, **dadurch gekennzeichnet, daß** die Klebefläche mindestens eines der Klebeetiketten zumindest teilweise von einer nichtklebenden Abdeckung (14) abgedeckt ist.

7. Packung nach Anspruch 6, **dadurch gekennzeichnet, daß** mindestens eine der Klebeetiketten (15a, 15b) eine vorzugsweise außerhalb der Abdeckung (14) angebrachte und zum Abtrennen eines zumindest einen Teil der Abdeckung aufweisenden Abschnitts des Klebeetikettes vom Rest des Klebeetikettes dienende Trennlinie, wie etwa eine Perforation (16) aufweist.

8. Packung nach einem der vorhergehenden Ansprüche, **dadurch gekennzeichnet, daß** die Außenverpackung eine tiefgezogene Schale (10) mit einer vorzugsweise aus einer aufgewickelten Papierbahn (11) bestehenden Abdeckung aufweist.

9. Packung nach einem der Ansprüche 1 - 8, **dadurch gekennzeichnet, daß** die Innenverpackung mindestens eine vorzugsweise im wesentlichen ebene Hauptfläche (110) aufweisenden und zum Bilden einer Entnahmeöffnung für das Sterilgut vorbereiteten Hülle (100), wobei die Hülle (100) zum Bilden der Entnahmeöffnung im Bereich der Hauptfläche (110) vorbereitet ist.

10. Packung nach Anspruch 9, **dadurch gekennzeichnet, daß** die Hülle (100) zum Bilden einer sich ausgehend von der Hauptfläche (110) bis über einen daran angrenzenden Rand des darin aufgenommenen Sterilgutes (140, 142, 144) hinweg erstreckenden Entnahmeöffnung vorbereitet ist.

11. Packung nach Anspruch 9 oder 10, **dadurch gekennzeichnet, daß** die Hülle (100) eine das Sterilgut zumindest teilweise umlaufende Materialbahn aufweist.

12. Packung nach Anspruch 11, **dadurch gekennzeichnet, daß** die Materialbahn einen ersten Abschnitt (101) und einen zweiten Abschnitt (102) aufweist, von denen jeder einen Teil der Hauptfläche (110) bildet, wobei sich diese Abschnitte (101, 102) im Bereich der Hauptfläche (110) zumindest teilweise überlappen.

13. Packung nach Anspruch 11 oder 12, **dadurch gekennzeichnet, daß** die Materialbahn einen ersten, einen Teil der Hauptfläche (110) bildenden Abschnitt (101), einen zweiten, einen weiteren Teil der Hauptfläche (110) bildenden Abschnitt (102), einen dritten, eine erste Umlaufachse teilweise umlaufenden Abschnitt (103), einen vierten, die der Hauptfläche (110) entgegengesetzte Begrenzungsfläche der Innenverpackung bildenden Abschnitt (104) und einen fünften, eine zweite, vorzugsweise etwa parallel zu der ersten Umlaufachse verlaufende Umlaufachse teilweise umlaufenden Abschnitt (105) aufweist.

14. Packung nach Anspruch 12 oder 13, **dadurch gekennzeichnet, daß** sich der erste Abschnitt (101) und der zweite Abschnitt (102) längs etwa parallel zu den Umlaufachsen verlaufenden Überlappungsbereich (112) überlappen.

15. Packung nach einem der Ansprüche 12 - 14, **dadurch gekennzeichnet, daß** der erste Abschnitt (101) und der zweite Abschnitt (102) in dem Überlappungsbereich (112) lösbar miteinander verbunden, insbesondere verklebt oder versiegelt sind.

16. Packung nach Anspruch 15, **dadurch gekennzeichnet, daß** die Haltekraft der Verbindung so gering ist, daß beim Lösen der Verbindung keine Beschädigung des ersten Abschnittes (101) oder zweiten Abschnittes (102) erfolgt.

17. Packung nach einem der Ansprüche 13 - 16, **dadurch gekennzeichnet, daß** der zweite Abschnitt (102) auf seiner dem Sterilgut (140, 142, 144) zugewandten Seite mit dem ersten Abschnitt (101) verbunden ist und in einer sich senkrecht zu den Umlaufachsen erstreckende Richtung eine die Hälfte der Breite der Hauptfläche (110) überschreitende Breite aufweist.

18. Packung nach einem der Ansprüche 12 - 17, **dadurch gekennzeichnet, daß** der erste Abschnitt (101) längs einer parallel zum Überlappungsbereich (112) verlaufenden Linie auf sich selbst zurückgefaltet ist und vorzugsweise in einer sich senkrecht zu den Umlaufachsen verlaufenden Richtung eine die Hälfte der Breite der Hauptfläche (110) überschreitende Breite aufweist.

19. Packung nach einem der Ansprüche 12 - 17, **dadurch gekennzeichnet, daß** ein freiliegender Rand (102a) des ersten Abschnittes (101) und/oder des zweiten Abschnittes (102) zur Bildung einer verstärkten Anfaßlasche zumindest teilweise auf sich selbst zurückgefaltet ist.

20. Packung nach einem der Ansprüche 13 - 19, **dadurch gekennzeichnet, daß** der erste Abschnitt (101) und/oder der zweite Abschnitt (102) längs mindestens eines quer zu den Umlaufachsen verlaufenden Verbindungsbereiches (120, 103) mit dem vierten Abschnitt (104) verbunden, insbesondere verklebt oder versiegelt ist, wobei die Haltekraft dieser Verbindung vorzugsweise so gering ist, daß beim Lösen der Verbindung keine Beschädigung des ersten, zweiten oder vierten Abschnittes (101, 102, 104) erfolgt.

21. Packung nach Anspruch 20 **dadurch gekennzeichnet, daß** sich die Verbindung des ersten Abschnittes (101) mit dem zweiten Abschnitt (102) nicht mit der Verbindung des ersten bzw. zweiten Abschnittes (101, 102) mit dem vierten Abschnitt (104) kreuzt.

22. Vorrichtung zum Herstellen einer Packung nach einem der vorhergehenden Ansprüche, mit einer Einrichtung zum Verschließen einer die sterile Innenverpackung (13) enthaltenden Außenverpackung (10, 11) und einer zum Befestigen eines Informationsträgers (15a) an der sterilen Innenverpackung (13) betreibbaren Befestigungseinrichtung (26), **dadurch gekennzeichnet, daß** die Befestigungseinrichtung (26) zum Befestigen von Informationsträgern (15b) an der ihrem Innenraum zugewandten Innenseite der Außenverpackung (11) betreibbar ist.

23. Vorrichtung nach Anspruch 22, **dadurch gekennzeichnet, daß** die Verschließeinrichtung eine erste zum Fördern der Außenverpackung längs einer vorgegebenen Bahn betreibbare Fördereinrichtung und eine zweite zum Fördern der Abdeckfolie für die Außenverpackung längs der vorgegebenen Bahn betreibbare Fördereinrichtung aufweist, wobei die zweite Fördereinrichtung eine zum Umlenken der Abdeckfolie um mindestens 90°, vorzugsweise etwa 180° um eine senkrecht zu der vorgegebenen Bahn verlaufende Umlenkachse dienende Umlenkeinrichtung (42) aufweist.

24. Vorrichtung nach Anspruch 22 oder 23, **dadurch gekennzeichnet, daß** die Befestigungseinrichtung eine zum Zuführen der Informationsträger in einer etwa senkrecht zur vorgegebenen Bahn verlaufenden Zuführrichtung (P) betreibbare Zuführeinrichtung sowie ein Befestigungselement, wie etwa einen in einer senkrecht zu der durch die Zuführrichtung (P) und die vorgegebene Bahn aufgespannten Ebene verlaufenden Aufdruckrichtung (DP) hin- und hergehend bewegbaren Stempel (26) aufweist.

25. Vorrichtung nach Anspruch 24, **dadurch gekennzeichnet, daß** die Zuführeinrichtung und/oder das Befestigungselement in einer parallel zu der vorgegebenen Bahn verlaufenden Richtung hin- und hergehend bewegbar sind.

26. Vorrichtung nach Anspruch 24 oder 25, **dadurch gekennzeichnet, daß** die Zuführeinrichtung zum Zuführen von auf einer nichtklebenden Trägerfolie angebrachten Klebeetiketten ausgelegt ist und mindestens eine Abzieheinrichtung zum Abziehen mindestens eines Randbereichs der Trägerfolie von den Klebeetiketten umfaßt.

27. Vorrichtung nach einem der Ansprüche 22 - 26 **dadurch gekennzeichnet, daß** die Befestigungseinrichtung zum gleichzeitigen Festlegen von zwei Informationsträgern auf zwei Innenverpackungen und/oder zwei Außenverpackungen betreibbar ist.

28. Vorrichtung nach einem der Ansprüche 23 - 27, **dadurch gekennzeichnet, daß** die erste Fördereinrichtung zum Fördern von mindestens zwei in einer senkrecht zu der vorgegebenen Bahn verlaufenden Richtung nebeneinander angeordneten Außenverpackungen ausgelegt ist.

29. Verfahren zum Herstellen einer Packung nach einem der Ansprüche 1 - 8 mit einer Vorrichtung nach einem der Ansprüche 22 - 28, bei dem eine Sterilgut enthaltende Innenverpackung in eine Außenverpackung eingelegt, ein Informationsträger an der in die Außenverpackung eingelegten Innenverpackung angebracht und die Außenverpackung verschlossen wird, **dadurch gekennzeichnet, daß** zusätzlich auch noch ein Informationsträger an der ihrem Innenraum zugewandten Innenseite der Außenverpackung angebracht wird.

## Revendications

1. Emballage pour stocker des produits stériles avec un emballage externe (10, 11) comprenant un intérieur stérile, un emballage interne (13) contenant les produits stériles et contenu dans l'intérieur stérile de l'emballage externe (10, 11) et au moins deux supports d'information (15a, 15b) comportant des informations concernant le contenu de l'emballage interne (13), dont au moins un (15a) est disposé dans l'intérieur stérile de l'emballage externe (10, 11), moyennant quoi au moins des supports d'information (15b) peut être retiré, par une personne non stérile, sans contamination de l'emballage interne (13) lors du processus de retrait de l'emballage interne (13) hors de l'emballage externe (10, 11), **caractérisé en ce que** les deux supports d'information (15a, 15b) sont disposés dans l'intérieur stérile de l'emballage externe (10, 11).

2. Emballage selon la revendication 1, **caractérisé en ce qu'**au moins un des supports d'information (15b) est fixé sur l'emballage externe (11).

3. Emballage selon la revendication 1 ou 2, **caractérisé en ce qu'**au moins un des supports d'information (15b) est fixé de manière amovible sur un côté interne de l'emballage externe (10, 11) orienté vers l'intérieur stérile d'une zone de recouvrement (11).

4. Emballage selon l'une des revendications précédentes, **caractérisé en ce qu'**au moins un des supports d'information (15a) est fixé de manière amovible sur un côté externe de l'emballage interne (13) stérile.

5. Emballage selon l'une des revendications précédentes, **caractérisé en ce qu'**au moins un des supports d'information (15a, 15b) présente une étiquette adhésive munie d'une impression (17) indiquant le contenu de l'emballage interne (13) stérile.

6. Emballage selon la revendication 5, **caractérisé en ce que** la surface adhésive d'au moins une des étiquettes adhésives est recouverte au moins partiellement d'un revêtement non adhésif (14).

7. Emballage selon la revendication 6, **caractérisé en ce qu'**au moins une des étiquettes adhésives (15a, 15b) comprend, une ligne de séparation, comme des perforations (16) disposée de préférence à l'extérieur, du revêtement (14) servant à séparer une partie de l'étiquette adhésive comprenant au moins une partie du revêtement du reste de l'étiquette adhésive.

8. Emballage selon l'une des revendications précédentes, **caractérisé en ce que** l'emballage externe comprend une coque emboutie (10) avec un revêtement constitué de préférence d'une bande de papier (11) enroulée.

9. Emballage selon l'une des revendications 1 à 8, **caractérisé en ce que** l'emballage interne comprend au moins une surface principale (110) globalement plane et un étui (100) préparer pour la formation d'une ouverture de retrait pour les produits stériles, moyennant quoi l'étui (100) est préparé à la formation de l'ouverture de retrait au niveau de la surface principale (110).

10. Emballage selon la revendication 9, **caractérisé en ce que** l'étui (100) est préparé pour la formation d'une ouverture de retrait qui s'étend de la surface principale (110) jusqu'au-dessus d'un bord adjacent des produits stériles (140, 142, 144) qui y sont contenus.

11. Emballage selon la revendication 9 ou 10, **caractérisé en ce que** l'étui (100) comprend une bande de matériau entourant au moins partiellement les produits stériles.

12. Emballage selon la revendication 11, **caractérisé en ce que** la bande de matériau comprend une première partie (101) et une deuxième partie (102), dont chacune constitue une partie de la surface principale (110), moyennant quoi ces parties (101, 102) se chevauchent au moins partiellement au niveau de la surface principale (110).

13. Emballage selon la revendication 11 ou 12, **caractérisé en ce que** la bande de matériau comprend une première partie (101) constituant une partie de la surface principale (110), une deuxième partie (1,02) constituant une autre partie de la surface principale (110), une troisième partie (103) entourant partiellement un premier axe de rotation, une quatrième partie (104) constituant la surface de limitation de l'emballage interne opposée à la surface principale (110) et une cinquième partie (105) entourant partiellement un deuxième axe de rotation de préférence approximativement parallèle au premier axe de rotation.

14. Emballage selon la revendication 12 ou 13, **caractérisé en ce que** la première partie (101) et la deuxième partie (102) se chevauchent le long d'une zone de chevauchement (112) approximativement parallèle aux axes de rotation.

15. Emballage selon l'une des revendications 12 à 14, **caractérisé en ce que** la première partie (101) et la deuxième partie (102) sont reliées de manière amovible entre elles, de préférence collées ou scellées, dans la zone de chevauchement (112).

16. Emballage selon la revendication 15, **caractérisé en ce que** la force de maintien est faible, de telle sorte que, lors du détachement de la liaison, aucun endommagement de la première partie (101) ou de la deuxième partie (102) ne survienne.

17. Emballage selon l'une des revendications 13 à 16, **caractérisé en ce que** la deuxième partie (102) est reliée avec la première partie (101) sur son côté orienté vers les produits stériles (140, 142, 144) et présente, dans une direction perpendiculaire aux axes de rotation, une largeur dépassant la moitié de la largeur de la surface principale (110).

18. Emballage selon l'une des revendications 12 à 17, **caractérisé en ce que** la première partie (101) est repliée sur elle-même le long d'une ligne parallèle à la zone de chevauchement (112) et présente de préférence, dans une direction perpendiculaire aux axes de rotation, une largeur dépassant la moitié de la largeur de la surface principale (110).

19. Emballage selon l'une des revendications 12 à 17, **caractérisé en ce qu'**un bord libre (102a) de la première partie (101) et/ou de la deuxième partie (102) est replié au moins partiellement sur lui-même pour former une patte de préhension renforcée.

20. Emballage selon l'une des revendications 13 à 19, **caractérisé en ce que** la première partie (101) et/ou la deuxième partie (102) est reliée, le long d'au moins une zone de liaison (120, 130) perpendiculaire aux axes de rotation, avec la quatrième partie (104), de préférence collée ou scellée, moyennant quoi la force de maintien de cette liaison soit de préférence faible, de façon à ce que, lors du détachement de cette liaison, aucun endommagement de la première, deuxième ou quatrième partie (101, 102, 104) ne survienne.

21. Emballage selon la revendication 20, **caractérisé en ce que** la liaison de la première partie (101) avec la deuxième partie (102) ne croise pas la liaison de la première ou deuxième partie (101, 102) avec la quatrième partie (104).

22. Dispositif de fabrication d'un emballage selon l'une des revendications précédentes, avec un dispositif de fermeture d'un emballage externe (10, 11) contenant l'emballage interne (13) stérile et un dispositif de fixation (26) conçu pour fixer un support d'information (15a) sur l'emballage interne (13) stérile, **caractérisé en ce que** le dispositif de fixation (26) est conçu pour fixer des supports d'informations (15b) sur le côté interne orienté vers l'intérieur de l'emballage externe (11).

23. Dispositif selon la revendication 22, **caractérisé en ce que** le dispositif de fermeture comprend un premier dispositif de transport conçu pour transporter l'emballage externe le long d'une trajectoire prédéterminée et un deuxième dispositif de transport conçu pour transporter le film de revêtement pour l'emballage externe le long de la trajectoire prédéterminée, moyennant quoi le deuxième dispositif de transport comprend un dispositif de déviation (42) pour dévier le film de revêtement d'au moins 90°, de préférence d'environ 180°, autour d'un axe de déviation perpendiculaire à al trajectoire prédéterminée.

24. Dispositif selon la revendication 22 ou 23, **caractérisé en ce que** le dispositif de fixation comprend un dispositif d'alimentation conçu pour alimenter les supports d'informations dans une direction d'alimentation (P) approximativement perpendiculaire à la trajectoire prédéterminée, ainsi qu'un élément de fixation, comme un poinçon (26) qui effectue un mouvement de va et vient dans une direction d'impression (DP) perpendiculaire au plan formé par le direction d'alimentation (P) et la trajectoire prédéterminée.

25. Dispositif selon la revendication 24, **caractérisé en ce que** le dispositif d'alimentation et/ou l'élément de fixation effectuent un mouvement de va et vient dans une direction parallèle à al trajectoire prédéterminée.

26. Dispositif selon la revendication 24 ou 25, **caractérisé en ce que** le dispositif d'alimentation est conçu pour alimenter des étiquettes adhésifs disposées sur film support non adhésif, et comprend au moins un dispositif de retrait pour retirer au moins un bord du film support des étiquettes adhésives.

27. de fixation est conçu pour la fixation simultanée de deux supports d'information sur deux emballages internes et/ou externes.

28. Dispositif selon l'une des revendications 23 à 27, **caractérisé en ce que** le premier dispositif de transport est conçu pour transporter au moins deux emballages externes disposés l'un à côté de l'autre dans une direction perpendiculaire à la trajectoire prédéterminée.

29. Procédé de fabrication d'un emballage selon l'une des revendications 1 à 8, avec un dispositif selon l'une des revendications 22 à 28, dans lequel un emballage interne contenant des produits stériles est inséré dans un emballage externe, un support d'information est fixé sur l'emballage interne inséré dans l'emballage externe et l'emballage externe est fermé, **caractérisé en ce qu'**un support d'information supplémentaire est fixé sur le côté interne de l'emballage externe orienté vers l'intérieur.

## Claims

1. Packaging for storing sterile goods, comprising an outer packaging (10, 11) having a sterile inner cavity, a sterile inner packaging (13) containing the sterile goods and contained in the sterile inner cavity of the outer packaging (10, 11), and at least two information-carrying information carriers (15a, 15b) displaying at least the contents of the inner packaging (13), at least one of which information carriers (15a) is arranged in the sterile inner cavity of the outer packaging (10, 11), at least one of the information carriers (15b) being able to be grasped by an unsterile person without contamination of the inner packaging (13) during the process of removing the inner packaging (13) from the outer packaging (10, 11), **characterised in that** both information carriers (15a, 15b) are arranged in the sterile inner cavity of the outer packaging (10, 11).

2. Packaging according to claim 1, **characterised in that** at least one of the information carriers (15b) is fixed to the outer packaging (11).

3. Packaging according to claim 1 or 2, **characterised in that** at least one of the information carriers (15b) is fixed detachably to an inner face of a covering area (11) of the outer packaging (10, 11), which inner face is oriented towards the sterile inner cavity.

4. Packaging according to any one of the preceding claims, **characterised in that** at least one of the information carriers (15a) is fixed detachably to an outer face of the sterile inner packaging (13).

5. Packaging according to any one of the preceding claims, **characterised in that** at least one of the information carriers (15a, 15b) has an adhesive label provided with a printed inscription (17) indicating the contents of the sterile inner packaging (13).

6. Packaging according to claim 5, **characterised in that** the adhesive surface of at least one of the adhesive labels is covered at least partially by a non-adhesive covering (14).

7. Packaging according to claim 6, **characterised in that** at least one of the adhesive labels (15a, 15b) has a dividing line, such as a perforation (16), located preferably outside the covering (14) and serving to allow a portion of the adhesive label carrying at least a part of the covering to be detached from the remainder of the adhesive label.

8. Packaging according to any one of the preceding claims, **characterised in that** the outer packaging has a recessed tray (10) with a cover preferably formed from a rolled strip of paper (11).

9. Packaging according to any one of claims 1 to 8, **characterised in that** the inner packaging has at least one sheath (100) having a preferably substantially flat main face (110) and prepared for forming a removal opening for removing the sterile goods, the sheath (100) being prepared for forming the removal opening in the area of the main face (110).

10. Packaging according to claim 9, **characterised in that** the sheath (100) is prepared for forming a removal opening starting from the main face (110) and extending beyond an edge of the sterile goods (140, 142, 144) contained therein, which edge is adjacent to the main face (110).

11. Packaging according to claim 9 or 10, **characterised in that** the sheath (100) has a strip of material extending at least partially around the sterile goods.

12. Packaging according to claim 11, **characterised in that** the strip of material has a first section (101) and a second section (102), each of which sections forms a part of the main face (110), these sections (101, 102) overlapping one another at least partially in the area of the main face (110).

13. Packaging according to claim 11 or 12, **characterised in that** the strip of material has a first section (101) forming one part of the main face (110), a second section (102) forming a further part of the main face (110), a third section (103) extending around a first axis of rotation, a fourth section (104) forming the boundary face of the inner packaging opposite the main face (110) and a fifth section (105) extending partially around a second axis of rotation disposed preferably approximately parallel to the first axis of rotation.

14. Packaging according to claim 12 or 13, **characterised in that** the first section (101) and the second section (102) overlap one another along an overlap area (112) disposed approximately parallel to the axes of rotation.

15. Packaging according to any one of claims 12 to 14, **characterised in that** the first section (101) and the second section (102) are detachably joined to one another, in particular glued or sealed, in the overlap area (112).

16. Packaging according to claim 15, **characterised in that** the adhesive strength of the join is so low that no damage is caused to the first section (101) or second section (102) when the join is separated.

17. Packaging according to any one of claims 13 to 16, **characterised in that** the second section (102) is joined to the first section (101) on its side facing towards the sterile goods (140, 142, 144) and has a width in a direction extending perpendicularly to the axes of rotation greater than half the width of the main face (110).

18. Packaging according to any one of claims 12 to 17, **characterised in that** the first section (101) is folded back on itself along a line extending parallel to the overlap area (112) and preferably has a width in a direction extending perpendicularly to the axes of rotation greater than half the width of the main face (110).

19. Packaging according to any one of claims 12 to 17, **characterised in that** a free edge (102a) of the first section (101) and/or of the second section (102) is folded back on itself at least partially to form a reinforced tongue for grasping.

20. Packaging according to any one of claims 13 to 19, **characterised in that** the first section (101) and/or the second section (102) is joined, in particular glued or sealed, to the fourth section (104) along at least one joining zone (120, 103) disposed transversely to the axes of rotation, the adhesive strength of this join preferably being so low that no damage is caused to the first, second or fourth section (101, 102, 104) when the join is separated.

21. Packaging according to claim 20, **characterised in that** the join of the first section (101) to the second section (102) does not cross the join of the first or second section (101, 102) to the fourth section (104).

22. Device for producing a packaging according to any one of the preceding claims, comprising an arrangement for closing an outer packaging (10, 11) containing the sterile inner packaging (13) and a fixing arrangement (26) operable for fixing an information carrier (15a) to the sterile inner packaging (13), **characterised in that** the fixing arrangement (26) is operable for fixing information carriers (15b) to the inner face of the outer packaging (11) which faces towards its inner cavity.

23. Device according to claim 22, **characterised in that** the closing arrangement has a first feed arrangement operable for feeding the outer packaging along a predefined path and a second feed arrangement operable for feeding the covering film for the outer packaging along the predefined path, the second feed arrangement having a guide device (42) serving to deflect the covering film through at least 90°, preferably approximately 180°, about an axis of deflection disposed perpendicularly to the predefined path.

24. Device according to claim 22 or 23, **characterised in that** the fixing arrangement has a feed arrangement operable for feeding the information carriers in a feed direction (P) extending approximately perpendicularly to the predefined path and a fixing element, such as a stamp (26) reciprocatingly movable in an application direction (DP) extending perpendicularly to the plane defined by the feed direction (P) and the predefined path.

25. Device according to claim 24, **characterised in that** the feed arrangement and/or the fixing element is reciprocatingly movable in a direction parallel to the predefined path.

26. Device according to claim 24 or 25, **characterised in that** the feed arrangement is arranged to feed adhesive labels affixed to a non-adhesive carrier film and includes at least one detaching device for detaching at least an edge portion of the carrier film from the adhesive labels.

27. Device according to any one of claims 22 to 26, **characterised in that** the fixing arrangement is operable for simultaneously fixing two information carriers on two inner packagings and/or two outer packagings.

28. Device according to any one of claims 23 to 27, **characterised in that** the first feed arrangement is arranged to feed at least two outer packagings arranged side-by-side in a direction extending perpendicularly to the predefined path.

29. Process for producing a packaging according to any one of claims 1 to 8, with a device according to any one of claims 22 to 28, in which an inner packaging containing an article of sterile goods is inserted in an outer packaging, an information carrier is affixed to the inner packaging inserted in the outer packaging and the outer packaging is closed, **characterised in that** an information carrier is additionally affixed to the inner face of the outer packaging which faces towards its inner cavity.
